(19) Europäisches Patentamt
European Patent Office
Office européen des brevets

(11) Publication number: **0 079 123**
**B1**

# (12) EUROPEAN PATENT SPECIFICATION

(45) Date of publication of patent specification: **04.09.85**

(51) Int. Cl.[4]: **C 07 D 455/06,** A 61 K 31/47

(21) Application number: **82305194.1**

(22) Date of filing: **30.09.82**

## (54) Benzoquinolizines.

(30) Priority: **07.10.81 GB 8130350**

(43) Date of publication of application:
**18.05.83 Bulletin 83/20**

(45) Publication of the grant of the patent:
**04.09.85 Bulletin 85/36**

(84) Designated Contracting States:
**AT BE CH DE FR IT LI LU NL SE**

(56) References cited:
**EP-A-0 047 105**
**US-A-4 076 820**
**US-A-4 183 937**

(73) Proprietor: **JOHN WYETH & BROTHER LIMITED**
**Huntercombe Lane South Taplow**
**Maidenhead Berkshire, SL6 0PH (GB)**

(72) Inventor: **Archibald, John Leheup Archibald**
**Koinonia Blackpond Lane**
**Farnham Royal Buckinghamshire (GB)**
Inventor: **Ward, Terence James**
**11, Iona Crescent**
**Cippenham Slough Berkshire (GB)**

(74) Representative: **Brown, Keith John Symons**
**et al**
**c/o John Wyeth & Brother Limited Patent and Trademark Department Huntercombe Lane South**
**Taplow Maidenhead Berkshire SL6 0PH. (GB)**

**EP 0 079 123 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to benzoquinolizines, to processes for preparing the benzoquinolizines and to pharmaceutical preparations containing them.

U.K. Patent Specification No. 1,513,824 discloses that benzoquinolizines of the general formula (I)

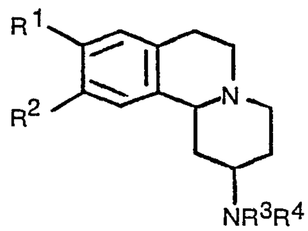

(I)

and the pharmaceutically acceptable acid addition salts thereof, wherein $R^1$ and $R^2$ which may be the same or different, each represent hydrogen, lower alkyl, lower alkoxy or halogen, $R^3$ represents hydrogen, lower alkyl or aryl and $R^4$ represents —$SO_2R^5$ (where $R^5$ is lower alkyl or aryl), —$CONH_2$ or —$CXNHR^6$ (where X is oxygen or sulphur and $R^6$ is aryl or aryl. CO.—), generally exhibit hypotensive activity upon administration to warm-blooded animals.

The specification of our U.K. Application No. 8125468 (published on 17th March 1982 under number 2083029A) discloses that benzoquinolizines of the general formula (II)

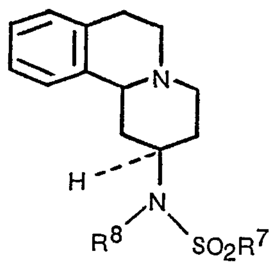

and the pharmaceutically acceptable acid addition salts thereof, wherein $R^7$ is lower alkyl or a phenyl or naphthyl group optionally substituted by one or more lower alkyl, lower alkoxy or halogen substituents and $R^8$ is methyl or ethyl possess presynaptic α-adrenoceptor antagonistic activity in warm blooded animals.

We have now found that N - methyl - N - (1,3,4,6,7,11bα - hexahydro - 2*H* - benzo[a]quinolizin - 2β - yl) - iso - butanesulphonamide, which is not disclosed specifically in either of the above mentioned specifications, together with its pharmaceutically acceptable acid addition salts, possesses extremely potent $\alpha_2$-adrenoceptor antagonistic activity and high presynaptic ($\alpha_2$) selectivity. Accordingly the present invention provides N - methyl - N - (1,3,4,6,7,11bα - hexahydro - 2*H* - benzo[a]quinolizin - 2β - yl) - *iso* - butanesulphonamide or a pharmaceutically acceptable acid addition salt thereof.

The presynaptic α-adrenoceptor antagonistic activity (or $\alpha_2$ antagonistic activity) of the compounds of the invention was investigated on the rat field stimulated vas deferens preparation using a modification of the method of Drew, Eur. J. Pharmac., 1977, *42*, 123—130. The procedure is described below.

Desheathed vasa deferentia from sexually mature rats were suspended in a 6 ml organ bath in Krebs solution at 37° and bubbled with 5% $CO_2$ in oxygen. Platinum ring electrodes were positioned above and below the tissue for field stimulation, the stimulus parameters being 0.1 Hz 1 ms pulse width at supramaximal voltage. Twitch responses were recorded isotonically with a 0.5 loading. Clonidine hydrochloride was used as the α-adrenoceptor agonist and cumulative concentration-response curves were constructed for the inhibition of twitch obtained with clonidine in the range 0.125 to 4 ng $ml^{-1}$. After washing out clonidine, the twitch response quickly recovered and an antagonist was then introduced into the Krebs reservoir. Clonidine concentration-response curves were repeated 90 min after introduction of the antagonist. The concentration of clonidine producing 50% inhibition of twitch before and after introduction of antagonist were obtained and the dose-ratio for clonidine was calculated. Various concentrations of the antagonists were used.

These results were plotted in the manner described by Arunlakshana & Schild, Br. J. Pharmac. Chemother., 1959, *14*, 48—58 and the values of $pA_2$ and slope were calculated. The compound of the invention possesses potent presynaptic α-adrenoceptor antagonistic ($\alpha_2$ antagonistic) activity having, a $pA_2$ value of 8.46 (95% confidence limits of 8.17—8.94), this value being higher than any of the values given for related compounds (including the isomeric *n*-butanesulphonamide) in the specification of U.K. Application No. 8125468.

The compound of the invention has been found to antagonise the presynaptic α-adrenoceptors to a much greater extent than the postsynaptic α-adrenoceptors. The postsynaptic antagonistic (or $\alpha_1$ antagonistic) activity can be evaluated by a number of different methods. One method involves assessing

the activity on the isolated anococcygeus muscle of the rat. The method is based on that of Gillespie, Br. J. Pharmac., 1972, *45*, 404—416. In the procedure male rats (250—360 g) are killed by a blow on the head and bled. The two anococcygeus muscles are removed from their position in the midline of the pelvic cavity, where they arise from the upper coccygeal vertebrae. The muscles are suspended in 5 ml organ baths in Krebs solution containing $10^{-4}$M ascorbic acid, to prevent drug oxidation. The tissues are gassed with a 95% oxygen, 5% $CO_2$ mixture and maintained at 37°. Longitudinal muscle contractions are recorded using isotonic transducers. Cumulative dose response curves are then obtained to phenylphrine or in some cases methoxamine, both agents being postsynaptic alpha adrenoceptor agonists. The concentration range of phenylephrine or methoxamine used is 0.02 to 0.8 $\mu g.ml^{-1}$. The agonist is then washed from the bath and the test drug added to the bathing medium at a concentration of $10^{-6}$M. After 30 min. equilibration with the test drug a further agonist dose response curve is obtained. The washing, equilibration and agonists dosing procedures are then repeated using $10^{-5}$M and $10^{-4}$M solutions of the test drug. Estimates of the $pA_2$ value for the test drug as an antagonist of phenylephrine or methoxamine were made from the agonist dose-ratios using the method of Arunlakshana & Schild, Br. J. Pharmac. Chemother., 1959, *14*, 48—58. The $pA_2$ for postsynaptic antagonistic activity for the compound of the invention was found to be 6.49 (with 95% confidence limits of 6.37—6.63). This means that the presynaptic selectivity ($pA_2$ presynaptic antagonistic activity/$pA_2$ postsynaptic antagonistic activity) was 93 which should be contrasted with a presynaptic selectivity of 19 for the isomeric n-butanesulphonamide disclosed in U.K. Application No. 8125468.

The compound of the invention has good presynaptic α-adrenoceptor antagonistic activity with high presynaptic selectivity and is of value in conditions where selective antagonism of the $\alpha_2$-adrenoceptor is desirable, for example, as an anti-depressant in treatment of diabetes and in inhibiting blood platelet aggregation. The compound of the invention has also been found to have 5-hydroxytryptamine (5-HT) antagonist activity. For example when tested in the rat isolated ileum the $pA_2$ for 5-HT antagonist activity was found to be 7.25.

The compounds of the present invention can be prepared by reacting a reactive derivative of isobutanesulphonic acid with 2β - methylamino - 1,3,4,6,7,11b - hexahydro - 2H - benzo[a]quinolizine and, if required, converting a free base into a pharmaceutically acceptable acid addition salt. The reactive derivative of the sulphonic acid can be, for example, the acid halide or anhydride. Preferably it is the halide e.g. isobutanesulphonyl chloride. The reaction is preferably carried out under basic conditions, for example in the presence of a tertiary amine, e.g. triethylamine.

In an alternative procedure the compounds of the invention can be prepared by catalytic hydrogenation of N - methyl - N - (1,3,4,6,7,11bα - hexahydro - 2*H* - benzo[a]quinolizin - 2β - yl) - 2 - methyl - 2 - propene - 1 - sulphonamide or an acid addition salt thereof and, if required, converting a free base into a pharmaceutically acceptable acid addition salt. The starting sulphonamide may be prepared by condensing 2β - methylamine - 1,3,4,6,7,11b - hexahydro - 2*H* - benzo[a]quinolizine with a reactive derivative of 2 - methylprop - 2 - ene - 1 - sulphonic acid, e.g., the sulphonyl chloride.

If in the processes described above the compound of the invention is obtained as an acid addition salt, the free base can be obtained by basifying a solution of the acid addition salt. Conversely, if the product of the process is a free base, an acid addition salt, particularly a pharmaceutically acceptable acid addition salt may be obtained by dissolving the free base in a suitable organic solvent and treating the solution with an acid, in accordance with conventional procedures for preparing acid addition salts from base compounds.

Examples of acid addition salts are those formed from inorganic and organic acids, such as sulphuric, hydrochloric, hydrobromic, phosphoric, tartaric, fumaric, maleic, citric, acetic, formic, methanesulphonic and *p*-toluenesulphonic acids.

The 2β - methylamino - 1,3,4,6,7,11b - hexahydro - 2H - benzo[a]quinolizine starting material can be prepared from the corresponding 2-oxo- compound by the procedure described in U.K. Patent Specification No. 1,513,824. Alternatively the 2-methylamino starting material can be prepared from the corresponding 2-amino compound, e.g. by reacting the amino compound with an alkylhaloformate or with formic acid and reducing, e.g. with a hydride transfer reagent such as lithium aluminium hydride, the resulting $2\text{-}NHCO_2Alkyl$ or 2-NHCHO intermediate.

The invention further provides a pharmaceutical composition comprising N - methyl - N - (1,3,4,6,7,11bα - hexahydro - 2*H* - benzo[a]quinolizin - 2β - yl) - *iso* - butane sulphonamide or a pharmaceutically acceptable acid addition salt thereof is association with a pharmaceutically acceptable carrier. Any suitable carrier known in the art can be used to prepare the pharmaceutical composition. In such a composition, the carrier is generally a solid or liquid or a mixture of a solid and a liquid.

Solid form compositions include powders, granules, tablets, capsules (e.g. hard and soft gelatin capsules), suppositories and pessaries. A solid carrier can be, for example, one or more substances which may also act as flavouring agents, lubricants, solubilisers, suspending agents, fillers, glidants, compression aids, binders or tablet-disintegrating agents; it can also be an encapsulating material. In powders the carrier is a finely divided solid which is in admixture with the finely divided active ingredient. In tablets the active ingredient is mixed with a carrier having the necessary compression properties in suitable proportions and compacted in the shape and size desired. The powders and tablets preferably contain up to 99%, e.g. from 0.03 to 99%, preferably 1 to 80% of the active ingredient. Suitable solid carriers include, for example, calcium phosphate, magnesium stearate, talc, sugars, lactose, dextrin, starch, gelatin, cellulose,

methyl cellulose, sodium carboxylmethyl cellulose, polyvinylpyrrolidine, low melting waxes and ion exchange resins.

The term "composition" is intended to include the formulation of an active ingredient with encapsulating material as carrier to give a capsule in which the active ingredient (with or without other carriers) is surrounded by the carrier, which is thus in association with it. Similarly cachets are included.

Liquid form compositions include, for example, solutions, suspensions, emulsions, syrups, elixirs and pressurised compositions. The active ingredient, for example, can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavouring agents, suspending agents, thickening agents, colours, viscosity regulators, stabilisers or osmo-regulators. Suitable examples of liquid carriers for oral and parenteral administration include water (particularly containing additives as above e.g. cellulose derivatives, preferably sodium carboxymethyl cellulose solution), alcohols (including monohydric alcohols and polyhydric alcohols e.g. glycerol and glycols) and their derivatives, and oils (e.g. fractionated coconut oil and arachis oil). For parenteral administration the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intravenously. When the compound is orally active it can be administered orally either in liquid or solid composition form.

Preferably the pharmaceutical composition is in unit dosage form, e.g., as tablets or capsules. In such form, the composition is sub-divided in unit doses containing appropriate quantities of the active ingredient; the unit dosage forms can be packaged compositions, for example packeted powders, vials, ampoules, prefilled syringes or sachets containing liquids. The unit dosage form can be, for example, a capsule or tablet itself, or it can be the appropriate number of any such compositions in package form. The quantity of the active ingredient in a dose of composition may be varied or adjusted from 0.5 mg or less to 750 mg or more, according to the particular need and the activity of the active ingredient. The invention also includes the compounds in the absence of the carrier where the compounds are in unit dosage form.

The following Example illustrates the invention:

Example 1

N - Methyl - N - (1,3,4,6,7,11bα - hexahydro - *2H* - benzo[a]quinolizin - 2β - yl) - isobutanesulphonamide

(a) iso-Butanesulphonic acid, sodium salt, was prepared by hydrogenation of commercially available 2 - methyl - 2 - propene - 1 - sulphonic acid, sodium salt, and converted to the sulphonyl chloride with $POCl_3$.

(b) An ice-cold, stirred solution of 2β - methylamino - 1,3,4,6,7,11bα - hexahydro - *2H* - benzo[a]quinolizine (2.16 g; 0.01M) and triethylamine (1.2 g; 0.012M) in dichloromethane (25 $cm^3$) was slowly treated with a solution of iso-butanesulphonyl chloride (1.57 g; 0.01M) in dichloromethane (25 $cm^3$). The clear solution was kept at room temperature for 6 days, washed with water (2×50 $cm^3$) and brine, dried ($MgSO_4$), filtered and evaporated to give a brown syrup (3.22 g). Chromatography on silica eluted with 10% ethanol-ethyl acetate gave a yellow oil (2.75 g) which was dissolved in hot ethanol (5 $cm^3$), acidified with ethanolic HCl, diluted with ethyl acetate (20 $cm^3$) and cooled. After about ½ hour, the crystals were filtered off, washed with 10% ethanol/ethyl acetate and dried at 80°/100 mm to give pure title compound (2.40 g) as colourless crystals, m.p. 210—212° (dec.).

**Claims for the Contracting States BE CH DE FR IT LI LU NL SE**

1. N - methyl - N - (1,3,4,6,7,11bα - hexahydro - *2H* - benzo[a]quinolizin - 2β - yl) - iso - butanesulphonamide or a pharmaceutically acceptable acid addition salt thereof.

2. A process for preparing a compound claimed in claim 1 which comprises reacting a reactive derivative of iso-butanesulphonic acid with 2β - methylamino - 1,3,4,6,7,11b - hexahydro - *2H* - benzo[a]quinolizine and, if required, converting a free base into a pharmaceutically acceptable acid addition salt.

3. A process for preparing a compound claimed in claim 1 which comprises catalytically hydrogenating N - methyl - N - (1,3,4,6,7,11bα - hexahydro - *2H* - benzo[a]quinolizin - 2β - yl) - 2 - methyl - 2 - propene - 1 - sulphonamide or an acid addition salt thereof and if required, converting a free base into a pharmaceutically acceptable acid addition salt.

4. A pharmaceutical composition having $\alpha_2$-adrenoceptor antagonistic activity comprising N - methyl - N - (1,3,4,6,7,11bα - hexahydro - *2H* - benzo[a]quinolizin - 2β - yl) - *iso* - butanesulphonamide or a pharmaceutically acceptable acid addition salt thereof in association with a pharmaceutically acceptable carrier.

5. N - Methyl - N - (1,3,4,6,7,11bα - hexahydro - *2H* - benzo[a]quinolizin - 2β - yl) - *iso* - butanesulphonamide or a pharmaceutically acceptable acid addition salt thereof for use in antagonising $\alpha_2$-adrenoceptors in warm blooded animals.

**Claims for the Contracting State AT**

1. A process for preparing N - methyl - N - (1,3,4,6,7,11bα - hexahydro - *2H* - benzo[a]quinolizin - 2β - yl) - *iso* - butanesulphonamide or a pharmaceutically acceptable acid addition salt thereof, which comprises reacting a reactive derivative of iso-butanesulphonic acid with 2β - methylamino - 1,3,4,6,7,11b - hexahydro - *2H* - benzo[a]quinolizine and, if required, converting a free base into a pharmaceutically acceptable acid addition salt.

2. A process for preparing N - methyl - N - (1,3,4,6,7,11bα - hexahydro - *2H* - benzo[a]quinolizin - 2β - yl) - iso - butanesulphonamide or a pharmaceutically acceptable salt thereof which comprises catalytically hydrogenating N - methyl - N - (1,3,4,6,7,11bα - hexahydro - *2H* - benzo[a]quinolizin - 2β - yl) - 2 - methyl - 2 - propene - 1 - sulphonamide or an acid addition salt thereof and if required, converting a free base into a pharmaceutically acceptable acid addition salt.

3. A process for preparing a pharmaceutically acceptable acid addition salt of N - methyl - N - (1,3,4,6,7,11bα - hexahydro - *2H* - benzo[a]quinolizin - 2β - yl) - *iso* - butanesulphonamide which comprises reacting the base with an inorganic or organic acid.

4. A process for preparing a pharmaceutical composition having $\alpha_2$-adrenoceptor antagonistic activity which comprises bringing N - methyl - N - (1,3,4,6,7,11bα - hexahydro - *2H* - benzo[a]quinolizin - 2β - yl) - *iso* - butanesulphonamide or a pharmaceutically acceptable acid addition salt thereof into association with a pharmaceutically acceptable carrier.

5. A process as claimed in claim 4 wherein the active ingredient is prepared by the process claimed in any one of claims 1 to 3.

**Patentansprüche für die Vertragsstaaten BE CH DE FR IT LI LU NL SE:**

1. N - Methyl - N - (1,3,4,6,7,11bα - hexahydro - 2H - benzo[a]chinolizin - 2β - yl) - isobutansulfonamid oder ein pharmazeutisch annehmbares Säureadditionssalz hievon.

2. Verfahren zum Herstellen einer Verbindung, wie in Anspruch 1 beansprucht, welches das Umsetzen eines reaktionsfähigen Derivats von Isobutansulfonsäure mit 2β - Methylamino - 1,3,4,6,7,11b - hexahydro - 2H - benzo[a]chinolizin und, wenn erforderlich, Überführen einer freien Base in ein pharmazeutisch annehmbares Säureadditionssalz umfaßt.

3. Verfahren zum Herstellen einer Verbindung, wie in Anspruch 1 beansprucht, welches das katalytische Hydrieren von N - Methyl - N - (1,3,4,6,7,11bα - hexahydro - 2H - benzo[a]chinolizin - 2β - yl) - 2 - methyl - 2 - propen - 1 - sulfonamid oder eines Säureadditionssalzes hievon und, wenn erforderlich, Überführen einer freien Base in ein pharmazeutisch annehmbares Säureadditionssalz umfaßt.

4. Pharmazeutische Zusammensetzung mit $\alpha_2$-adrenozeptorantagonistischer Aktivität, die N - Methyl - N - (1,3,4,6,7,11bα - hexahydro - 2H - benzo[a]chinolizin - 2β - yl) - isobutansulfonamid oder ein pharmazeutisch annehmbares Säureadditionssalz hievon in Vereinigung mit einem pharmazeutisch annehmbaren Träger aufweist.

5. N - Methyl - N - (1,3,4,6,7,11bα - hexahydro - 2H - benzo[a]chinolizin - 2β - yl) - isobutansulfonamid oder ein pharmazeutisch annehmbares Säureadditionssalz hievon zur Verwendung beim Antagonisieren von $\alpha_2$-Adrenozeptoren in Warmblütern.

**Patentansprüche für den Vertragsstaat AT**

1. Verfahren zum Herstellen von N - Methyl - N - (1,3,4,6,7,11bα - hexahydro - 2H - benzo[a]chinolizin - 2β - yl) - isobutansulfonamid oder eines pharmazeutisch annehmbaren Säureadditionssalzes hievon, welches das Umsetzen eines reaktionsfähigen Derivates von Isobutansulfonsäure mit 2β - Methylamino - 1,3,4,6,7,11b - hexahydro - 2H - benzo[a]chinolizin und, wenn gewünscht, Überführen einer freien Base in ein pharmazeutisch annehmbares Säureadditionssalz umfaßt.

2. Verfahren zum Herstellen von N - Methyl - N - (1,3,4,6,7,11bα - hexahydro - 2H - benzo[a]chinolizin - 2β - yl) - isobutansulfonamid oder eines pharmazeutisch annehmbaren Salzes hievon, welches das katalytische Hydrieren von N - Methyl - N - (1,3,4,6,7,11bα - hexahydro - 2H - benzo[a]chinolizin - 2β - yl) - 2 - methyl - 2 - propen - 1 - sulfonamid oder eines Säureadditionssalzes hievon und, wenn erforderlich, Überführen einer freien Base in ein pharmazeutisch annehmbares Säureadditionssalz umfaßt.

3. Verfahren zum Herstellen eines pharmazeutisch annehmbaren Säureadditionssalzes von N - Methyl - N - (1,3,4,6,7,11bα - hexahydro - 2H - benzo[a]chinolizin - 2β - yl) - isobutansulfonamid, welches das Umsetzen der Base mit einer anorganischen oder organischen Säure umfaßt.

4. Verfahren zum Herstellen einer pharmazeutischen Zusammensetzung mit $\alpha_2$-adrenzozeptor-antagonistischer Aktivität, welches das Vereinigen von N - Methyl - N - (1,3,4,6,7,11bα - hexahydro - 2H - benzo[a]chinolizin - 2β - yl) - isobutansulfonamid oder eines pharmazeutisch annehmbaren Säureadditionssalzes hievon mit einem pharmazeutisch annehmbaren Träger umfaßt.

5. Verfahren, wie in Anspruch 4 beansprucht, worin der aktive Bestandteil nach dem in einem der Ansprüche 1 bis 3 beanspruchten Verfahren hergestellt wird.

**Revendications pour les Etats Contractants BE CH DE FR IT LI LU NL SE**

1. Le N - méthyl - N - (1,3,4,6,7,11bα - hexahydro - *2H* - benzo[a]quinolizine - 2β - yl) - *iso* - butanesulfamide ou un sel d'addition d'acide pharmaceutiquement acceptable de ce composé.

2. Procédé de préparation d'un composé suivant la revendication 1, qui consiste à faire réagir un dérivé réactif d'acide isobutanesulfonique avec la 2β - méthylamino - 1,3,4,6,7,11b - hexahydro - *2H* - benzo[a]quinolizine, et, le cas échéant, à convertir une base libre en un sel d'addition d'acide pharmaceutiquement acceptable.

3. Procédé de préparation d'un composé suivant la revendication 1, qui consiste à hydrogéner catalytiquement le N - méthyl - N - (1,3,4,6,7,11bα - hexahydro - 2*H* - benzo[a]quinolizine - 2β - yl) - 2 - méthyl - 2 - propène - 1 - sulfamide ou un sel d'addition d'acide de ce composé et, les cas échéant, à convertir une base libre en un sel d'addition d'acide pharmaceutiquement acceptable.

4. Composition pharmaceutique douée d'activité antagoniste envers les récepteurs $\alpha_2$-adrénergiques, comprenant du N - méthyl - N - (1,3,4,6,7,11bα - hexahydro - 2*H* - benzo[a]quinolizine - 2β - yl) - *iso* - butanesulfamide ou un sel d'addition d'acide pharmaceutiquement acceptable de ce composé en association avec un support acceptable du point de vue pharmaceutique.

5. Le N - méthyl - N - (1,3,4,6,7,11bα - hexahydro - 2*H* - benzo[a]quinolizine - 2β - yl) - *iso* - butanesulfonamide ou un sel d'addition d'acide pharmaceutiquement acceptable de ce composé destiné à être utilisé pour antagoniser des récepteurs $\alpha_2$-adrénergiques chez des animaux à sang chaud.

**Revendications pour l'Etat Contractant AT**

1. Procédé de préparation du N - méthyl - N - (1,3,4,6,7,11bα - hexahydro - 2*H* - benzo[a]quinolizine - 2β - yl) - *iso* - butanesulfamide ou d'un sel d'addition d'acide pharmaceutiquement acceptable de ce composé, qui consiste à faire réagir un dérivé réactif de l'acide iso-butanesulfonique avec la 2β - méthylamino - 1,3,4,6,7,11b - hexahydro - 2*H* - benzo[a]quinolizine et, le cas échéant, à convertir une base libre en un sel d'addition d'acide pharmaceutiquement acceptable.

2. Procédé de préparation d'un N - méthyl - N - (1,3,4,6,7,11bα - hexahydro - 2*H* - benzo[a]quinolizine - 2β - yl) - iso - butanesulfamide ou d'un sel pharmaceutiquement acceptable de ce composé qui consiste à hydrogéner catalytiquement du N - méthyl - N - (1,3,4,6,7,11bα - hexahydro - 2*H* - benzo[a]quinolizine - 2β - yl) - 2 - méthyl - 2 - propène - 1 - sulfamide ou un sel d'addition d'acide de ce composé et, le cas échéant, à convertir une base libre en un sel d'addition d'acide pharmaceutiquement acceptable.

3. Procédé de préparation d'un sel d'addition d'acide pharmaceutiquement acceptable du N - méthyl - N - (1,3,4,6,7,11bα - hexahydro - 2*H* - benzo[a]quinolizine - 2β - yl) - *iso* - butanesulfamide, qui consiste à faire réagir la base avec un acide inorganique ou organique.

4. Procédé de préparation d'une composition pharmaceutique douée d'activité antagoniste envers les récepteurs $\alpha_2$-adrénergiques, qui consiste à mettre en association du N - méthyl - N - (1,3,4,6,7,11bα - hexahydro - 2*H* - benzo[a]quinolizine - 2β - yl) - *iso* - butanesulfamide ou un sel d'addition d'acide pharmaceutiquement acceptable de ce composé avec un support acceptable du point de vue pharmaceutique.

5. Procédé suivant la revendication 4, dans lequel l'ingrédient actif est préparé par le procédé suivant l'une quelconque des revendications 1 à 3.